# EUROPEAN PATENT APPLICATION

(11) **EP 2 415 497 A1**
(43) Date of publication of application: **08.02.2012**
(21) Application number: 11173676.5
(22) Date of filing: 12.07.2011
(51) Int. Cl.: A61M 25/09

(54) **Guidewire**

(30) Priority: 02.08.2010 JP 2010173603
(71) Applicant: ASAHI INTECC CO., LTD., Nagoya-shi, Aichi 463-0024 (JP)
(72) Inventor: Kanazawa, Yuuya, Nagoya-shi, Aichi 463-0024 (JP)
(74) Representative: Winter, Brandl, Fürniss, Hübner, Röss, Kaiser, Polte - Partnerschaft

(57) **Abstract**

A guidewire (1) includes a core shaft (2) and a coil body (3) wound around an outer periphery of the core shaft. A distal end portion of the core shaft and a distal end portion of the coil body are fixed to each other with a most distal end portion (4). The coil outer diameter of the coil body in the most distal end portion is nonuniform.

## Description

### BACKGROUND OF THE INVENTION

### 1. Field of the Invention

The present invention relates to a guidewire.

### 2. Description of the Related Art

To date, various guidewires have been proposed for guiding a medical device to a target region through a body tissue or a tubular organ, such as a blood vessel, an alimentary canal, or a ureter.

For example, Japanese Unexamined Patent Application Publication No. 2003-52829 describes a guidewire having a fixed portion formed at the distal end of a core wire or the like, the fixed portion preventing accidental removal of a fixing portion. As another example, the specification of U.S. Patent No. 5345945 describes a guidewire having a double-coil structure including an outer coil body and an inner coil body.

### SUMMARY OF THE INVENTION

The guidewire described in Japanese Unexamined Patent Application Publication No. 2003-52829, which has a fixed portion at the distal end of the core wire, is capable of preventing accidental removal of the fixing portion (brazed portion at the distal end). However, if such a fixed portion, which is capable of preventing accidental removal of the fixing portion, is provided to the core wire, the distal end of the guidewire may become too hard and the guidewire may damage a blood vessel or the like.

The guidewire described in U.S. Patent No. 5345945, which has a double-coil structure, is not capable of preventing accidental removal of a brazed distal end portion because the outer diameters of the two coil bodies are uniform.

The present invention has the object to provide a guidewire that prevents accidental removal of a most distal end portion of the guidewire while maintaining flexibility of a distal end portion of the guidewire. This object is solved by a guidewire according to claim 1. Preferred embodiments are subject-matter of dependent claims.

According to a first aspect of the invention, a guidewire includes a core shaft; a coil body wound around an outer periphery of the core shaft; and a most distal end portion formed by fixing a distal end portion of the core shaft and a distal end portion of the coil body to each other, wherein the coil outer diameter of the coil body in the most distal end portion is nonuniform.

According to a second aspect of the invention, a guidewire includes a core shaft; an outer coil body wound around an outer periphery of the core shaft; an inner coil body located inside the outer coil body and wound around an outer periphery of the core shaft; and a most distal end portion formed by fixing a distal end portion of the core shaft, a distal end portion of the outer coil body, and a distal end portion of the inner coil body to one another, wherein at least one of a coil outer diameter of the outer coil body and a coil outer diameter of the inner coil body in the most distal end portion is nonuniform.

The guidewire according to the first aspect of the invention has an advantage in that accidental removal of the most distal end portion of the guidewire is prevented, because the coil outer diameter of the coil body in the most distal end portion is nonuniform. Moreover, the guidewire has an advantage in that flexibility of the distal end portion of the guidewire is maintained and damage to a blood vessel or the like is prevented, because only the coil outer diameter of the coil body is made nonuniform irrespective of the shape of the core shaft.

The guidewire according to the second aspect of the invention has an advantage in that accidental removal of the most distal end portion of the guidewire is prevented because at least one of the coil outer diameter of the outer coil body and the coil outer diameter of the inner coil body is nonuniform. Moreover, the guidewire has an advantage in that flexibility of the distal end portion of the guidewire is maintained and damage to a blood vessel or the like is prevented, because only the coil outer diameter of the coil body is made nonuniform irrespective of the shape of the core shaft.

### BRIEF DESCRIPTION OF THE DRAWINGS

Fig. 1 illustrates an overall view of a guidewire according to a first embodiment of the present invention.

Fig. 2 illustrates an overall view of a guidewire according to a second embodiment of the present invention.

Fig. 3 illustrates an overall view of a guidewire according to a third embodiment of the present invention.

Fig. 4 illustrates an enlarged view of a distal end of a guidewire according to a first modification of the present invention.

Fig. 5 illustrates an enlarged view of a distal end of a guidewire according to a second modification of the present invention.

Fig. 6 illustrates an enlarged view of a distal end of a guidewire according to a third modification of the present invention.

Fig. 7 illustrates an enlarged view of a distal end of a guidewire according to a fourth modification of the present invention.

Fig. 8 illustrates an enlarged view of a distal end of a guidewire according to a fifth modification of the present invention.

Fig. 9 illustrates an enlarged view of a distal end of a guidewire according to a sixth modification of the present invention.

Figs. 10A and 10B each illustrate an enlarged view of a distal end of a guidewire according to a seventh modification of the present invention.

Fig. 11 illustrates an enlarged view of a distal end of a guidewire according to an eighth modification of the present invention.

Figs. 12A and 12B illustrate enlarged views of a distal end of a guidewire according to a ninth modification of the present invention.

### DESCRIPTION OF THE PREFERRED EMBODIMENTS

Hereinafter, guidewires according to embodiments of the present invention will be described with reference to the drawings.

### First Embodiment

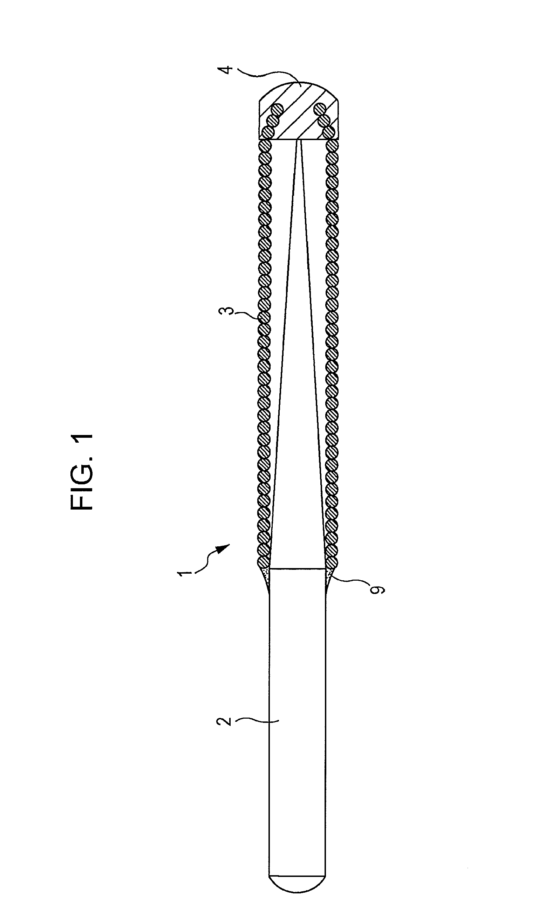

Fig. 1 illustrates an overall view of a guidewire 1 according to a first embodiment of the present invention.

For convenience of description, the left side in Fig. 1 will be referred to as "proximal end" and the right side will be referred to as "distal end". For ease of understanding, the length of the guidewire 1 is reduced and the guidewire 1 is schematically illustrated in Fig. 1. Therefore, the actual dimensions of the guidewire 1 differ from those of Fig. 1.

Referring to Fig. 1, the guidewire 1 includes a core shaft 2 and a coil body 3 that covers a distal end portion of the core shaft 2. The distal end portion of the core shaft 2 and a distal end portion of the coil body 3 are fixed to each other and form a most distal end portion 4. A proximal end portion of the coil body 3 is fixed to the core shaft 2 with a brazed joint 9 at a position between the most distal end portion 4 and a proximal end of the core shaft 2.

The material of the core shaft 2 is not particularly limited. For example, a stainless steel (SUS304), a superelastic alloy such as a Ni-Ti alloy, a piano wire, or the like can be used.

The materials of the most distal end portion 4 and the brazed joint 9, which fix the core shaft 2 and the coil body 3 to each other, are not particularly limited. For example, an aluminum brazing alloy, silver solder, gold solder, zinc solder, a Sn-Pb brazing alloy, a Pb-Ag brazing alloy, a Sn-Ag brazing alloy, or the like can be used.

It is preferable that the material of the most distal end portion 4 be a radiopaque material. By making the most distal end portion 4 from a radiopaque material, the position of the most distal end portion 4 can be detected by radiography.

The coil body 3 is a single wire coil made of a single strand. The coil outer diameter of the coil body 3 in the most distal end portion 4 decreases in the distal direction.

With such a structure, accidental removal of the most distal end portion 4 of the guidewire 1 from the guidewire 1 is prevented even if the most distal end portion 4 is trapped by a stenosis in a blood vessel or the like when pulling out the guidewire 1. This structure for preventing accidental removal is obtained by only changing the coil outer diameter of the coil body 3, so that the flexibility of a part of the guidewire 1 near the distal is maintained.

In the present embodiment, the most distal end portion 4 has a semispherical shape that is oriented in the distal direction. In the case where the most distal end portion 4 is made to have such a shape, the coil outer diameter of a part of the coil body 3 in the most distal end portion 4 may decrease in the distal direction as in the present embodiment.

By thus decreasing the coil outer diameter of the coil body 3, the distal end of the coil body 3 is prevented from protruding from the most distal end portion 4. Moreover, the distal end of the coil body 3 is located near the distal end of the most distal end portion 4, whereby accidental removal of the most distal end portion 4 from the guidewire 1 is more reliably prevented.

Examples of methods for making the coil body 3 include a method of forming a coil shape by winding a strand around a core bar having a decreasing outer diameter portion, and a method of pressing a distal end of a coil body having a uniform outer diameter into a die having an inverted cone shape to decrease the outer diameter of the distal end of the coil body. The method of making the coil body 3 is not limited to these examples. The coil body 3 may be made by using other known methods.

A radiopaque strand or a radiotransparent strand can be used as the material of the coil body 3. The material of the radiopaque strand is not particularly limited. For example, gold, platinum, tungsten, or an alloy of such metals (for example, a platinum-nickel alloy) can be used. The material of the radiotransparent strand is not particularly limited. For example, a stainless steel (SUS304, SUS316, or the like), a superelastic alloy such as a Ni-Ti alloy, a piano wire, or the like can be used.

The coil body 3 may be made radiopaque by forming a proximal end side of the coil body 3 from a radiotransparent strand and forming a distal end side of the coil body 3 from a radiopaque strand. In this case, the positional relationship between the coil body 3 and the most distal end portion 4 can be observed by radiography.

### Second Embodiment

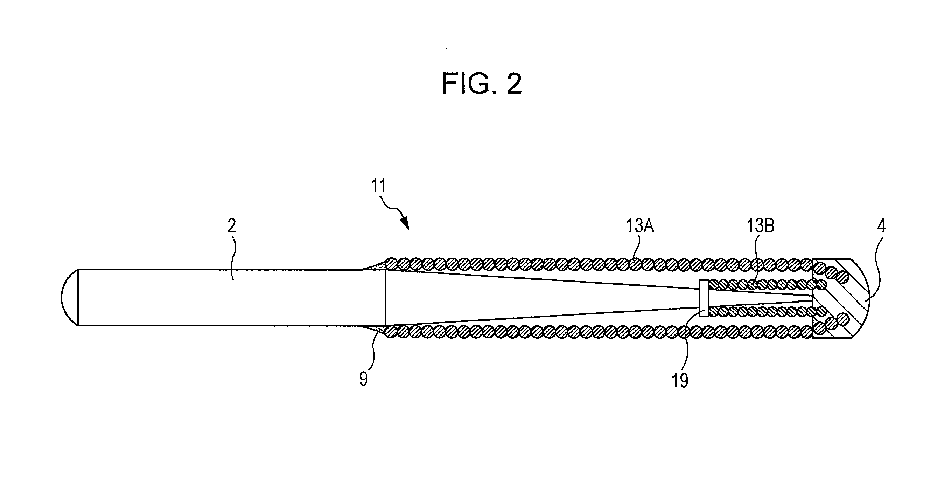

Referring to Fig. 2, a guidewire 11 according to a second embodiment will be described with emphasis on differences between the second embodiment and the first embodiment. Components of the second embodiment the same as those of the first embodiment will be denoted by the same numerals in Fig. 2. As in Fig. 1, for ease of understanding, the length of the guidewire 11 is reduced and the guidewire 11 is schematically illustrated in Fig. 2. Therefore, the actual dimensions of the guidewire 11 differ from those of Fig. 2.

The guidewire 11 includes the core shaft 2, an outer coil body 13A wound around an outer periphery of the core shaft 2, and an inner coil body 13B located inside the outer coil body 13A and wound around the outer periphery of the core shaft 2. A distal end portion of the core shaft 2, a distal end portion of the outer coil body 13A, and a distal end portion of the inner coil body 13B are fixed to one another and form the most distal end portion 4. A proximal end portion of the outer coil body 13A is fixed to the core shaft 2 with the brazed joint 9 at a position between the most distal end portion 4 and a proximal end of the core shaft 2. A proximal end portion of the inner coil body 13B is fixed to the core shaft 2 with a brazed joint 19 at a position between the most distal end portion 4 and the proximal end of the core shaft 2.

The coil outer diameter of a part of the outer coil body 13A in the most distal end portion 4 decreases in the distal direction. The coil outer diameter of a part of the inner coil body 13B in the most distal end portion 4 is the same as the coil outer diameter of a part of the inner coil body 13B that is located in the proximal direction of the most distal end portion 4.

The guidewire 11 has a double structure including the outer coil body 13A and the inner coil body 13B. Moreover, the coil outer diameter of a part of the outer coil body 13A in the most distal end portion 4 decreases in the distal direction. Therefore, accidental removal of the most distal end portion 4, which is disposed at the distal end of the guidewire 11, from the guidewire 11 is prevented. Moreover, because the guidewire 11 has a simple structure, in which the coil outer diameter of a part of the outer coil body 13A in the most distal end portion 4 decreases, the flexibility of the guidewire 11 is maintained.

The materials of the outer coil body 13A and the inner coil body 13B may be the same as that of the coil body 3 of the first embodiment. Although this is not a limitation, at least one of the outer coil body 13A and the inner coil body 13B may be a radiopaque coil body made of a radiopaque strand.

The coil outer diameter of the inner coil body 13B is smaller than that of the outer coil body 13A. By making the inner coil body 13B from a strand having a smaller diameter than the strand of the outer coil body 13A, the inner coil body 13B having a coil outer diameter smaller than that of the outer coil body 13A can be easily manufactured.

The material of the brazed joint 19 may be the same as the material of the most distal end portion 4 and the brazed joint 9.

### Third Embodiment

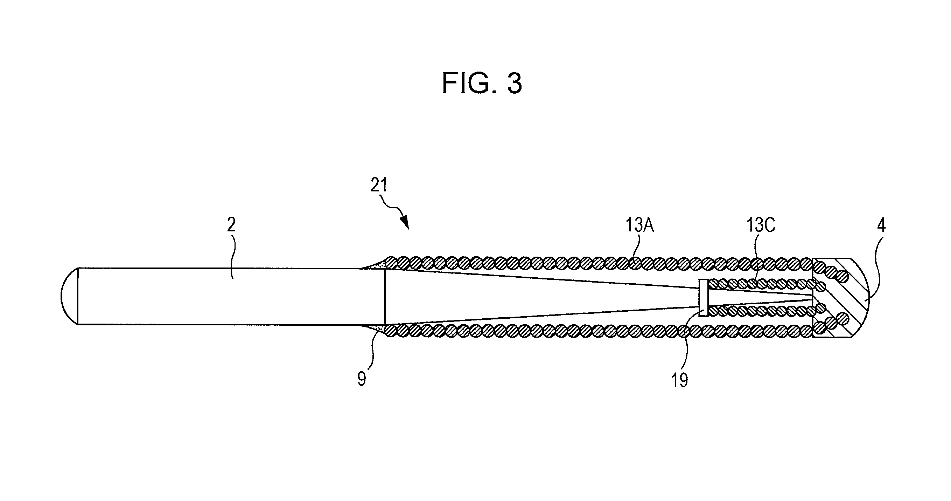

Referring to Fig. 3, a guidewire 21 according to a third embodiment will be described with emphasis on differences between the third embodiment and the second embodiment. Components of the third embodiment the same as those of the second embodiment will be denoted by the same numerals in Fig. 3. As in Fig. 1, for ease of understanding, the length of the guidewire 21 is reduced and the guidewire 21 is schematically illustrated in Fig. 3. Therefore, the actual dimensions of the guidewire 21 differ from those of Fig. 3.

The guidewire 21 includes an inner coil body 13C, and the coil outer diameter of a part of the inner coil body 13C in the most distal end portion 4 decreases in the distal direction. A proximal end portion of the inner coil body 13C is fixed to the core shaft 2 with the brazed joint 19 at a position in the proximal direction of the most distal end portion 4.

Thus, the guidewire 21 has a double structure including the outer coil body 13A and the inner coil body 13C, the coil outer diameter of a part of the outer coil body 13A in the most distal end portion 4 decreases in the distal direction, and the coil outer diameter of a part of the inner coil body 13C in the most distal end portion 4 decreases in the distal direction. As a result, accidental removal of the most distal end portion 4 of the guidewire 21 from the guidewire 21 is further reliably prevented. Moreover, because the guidewire 21 has a simple structure, in which the coil outer diameter of a part of the outer coil body 13A in the most distal end portion 4 and the coil outer diameter of a part of the inner coil body 13C in the most distal end portion 4 decrease, flexibility of the guidewire 21 is maintained.

The present invention is not limited to the above-described embodiments, and can be modified within the technical scope of the present invention by a person having an ordinary skill in the art. Hereinafter, each of modifications of the present invention will be described with reference to an enlarged view of a distal end of a guidewire. For ease of understanding, the length of a guidewire according to each modification is reduced and the guidewire is schematically illustrated in a corresponding enlarged view of the distal end thereof. Therefore, the actual dimensions of the guidewire differ from those illustrated in the figure.

### First Modification

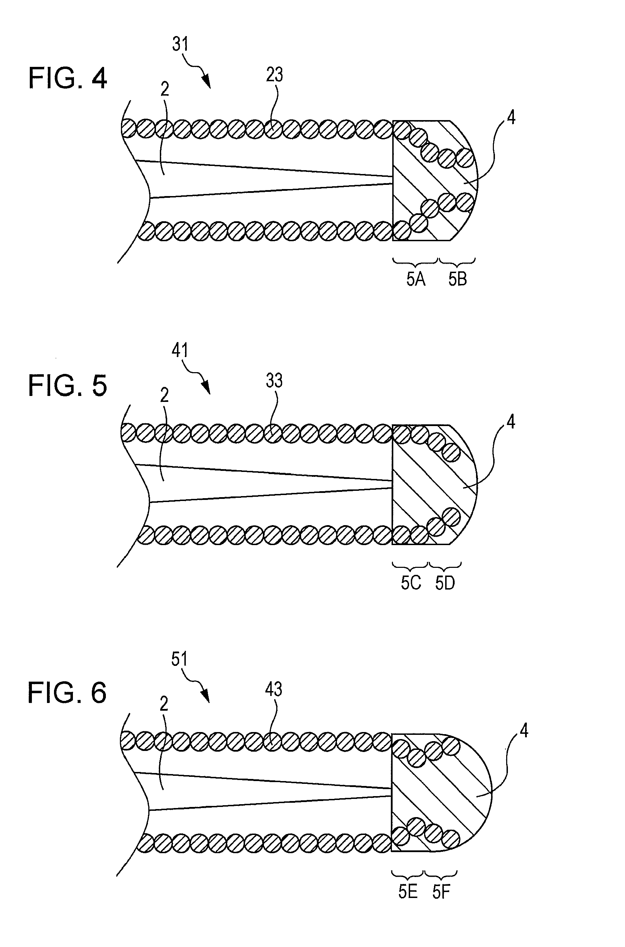

Fig. 4 illustrates a guidewire 31 including a coil body 23 whose coil outer diameter in the most distal end portion 4 is nonuniform. The coil body 23 has a decreasing-diameter portion 5A and a uniform-diameter portion 5B in the most distal end portion 4. The coil outer diameter of the decreasing-diameter portion 5A decreases from the proximal end of the most distal end portion 4 in the distal direction. The uniform-diameter portion 5B is disposed adjacent to the distal end of the decreasing-diameter portion 5A.

### Second Modification

Fig. 5 illustrates a guidewire 41 including a coil body 33 having a uniform-diameter portion 5C and a decreasing-diameter portion 5D in the most distal end portion 4. The uniform-diameter portion 5C extends from the proximal end of the most distal end portion 4 in the distal direction and has a uniform coil outer diameter. The decreasing-diameter portion 5D is disposed adjacent to the distal end of the uniform-diameter portion 5C and has a coil outer diameter that decreases in the distal direction.

The coil bodies 23 and 33, respectively according to the first modification and the second modification, can be made by winding a strand around, for example, a core bar having a decreasing diameter portion and a uniform diameter portion that are arranged, when making the coil body 23, in this order in the distal direction (when making the coil body 33, the configuration of the outer diameter of the core bar is opposite to this).

### Third Modification

Fig. 6 illustrates a guidewire 51 including a coil body 43 having a decreasing-diameter portion 5E and an increasing-diameter portion 5F in the most distal end portion 4. The decreasing-diameter portion 5E extends from the proximal end of the most distal end portion 4 and has a coil outer diameter that decreases in the distal direction. The increasing-diameter portion 5F is disposed adjacent to the distal end of the decreasing-diameter portion 5E and has a coil outer diameter that increases in the distal direction.

The coil body 43 can be made by, for example, preparing a coil body having a decreasing diameter and a bar having a tapered shape and having a distal end that is thinner than the coil inside diameter of the coil body and then inserting the bar into the coil body in the proximal direction from the distal end of the coil body.

### Fourth Modification

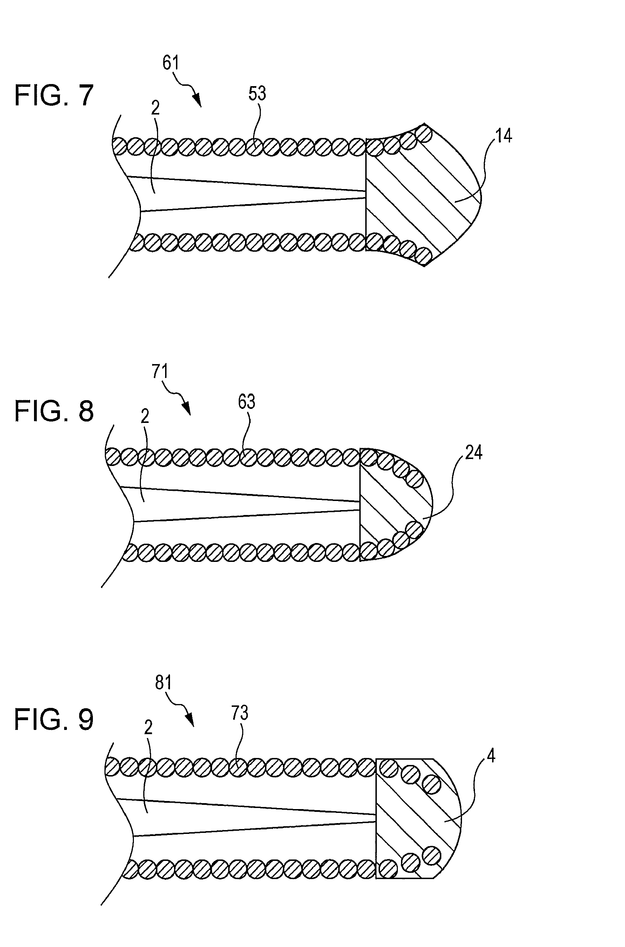

Fig. 7 illustrates a guidewire 61 including a coil body 53 having a coil outer diameter that does not decrease but gradually increases in the distal direction in a most distal end portion 14.

When the guidewire 61, which includes the coil body 53 having such a shape, is used to treat a stenosis in a blood vessel or the like, the most distal end portion 14 widens the passage through a stenotic site while the guidewire passes through the stenotic site, whereby contact between the stenotic site and a part the coil body 53 located in the proximal direction of the most distal end portion 14 is reduced. As a result, the operability of the guidewire in the stenotic site is maintained.

Moreover, the outer diameter of the coil body 53 in the most distal end portion 14 gradually increases from the coil outer diameter of the coil body 53 at the proximal end of the most distal end portion 14. Therefore, when the guidewire 61 is pulled back from the stenotic site, through which the guidewire 61 has passed, the most distal end portion 14 is not easily snagged on the stenotic site, and accidental removal of the most distal end portion 14 is prevented.

### Fifth Modification

Fig. 8 illustrates a guidewire 71 including a coil body 63 having a coil outer diameter that decreases in the distal direction in a most distal end portion 24. Moreover, the outer diameter of the most distal end portion 24 decreases in the distal direction so as to correspond to the decreasing coil outer diameter of the coil body 63.

By thus decreasing the outer diameter of the most distal end portion 24 in the distal direction, ease of insertion of the guidewire 71 into a peripheral portion of a blood vessel or the like is improved.

### Sixth Modification

Fig. 9 illustrates a guidewire 81 including a coil body 73 having a loosely-wound portion (having gaps between adjacent turns of a strand) in the most distal end portion 4, in which the coil outer diameter of the coil body 73 decreases. In this case, a brazing alloy or the like, of which the most distal end portion 4 is formed, can be easily made to flow into the coil body 73, whereby the most distal end portion 4 can be easily formed. Moreover, because the brazing alloy easily enters spaces between turns of the strands, the coil body 73 and the most distal end portion 4 can be fixed to each other firmly.

The configuration of the loosely-wound portion of the coil body 73 is not limited to that of this modification. The loosely-wound portion may be formed in a part of the coil body 73 in the most distal end portion 4, in which the coil outer diameter of the coil body 73 decreases.

### Seventh Modification

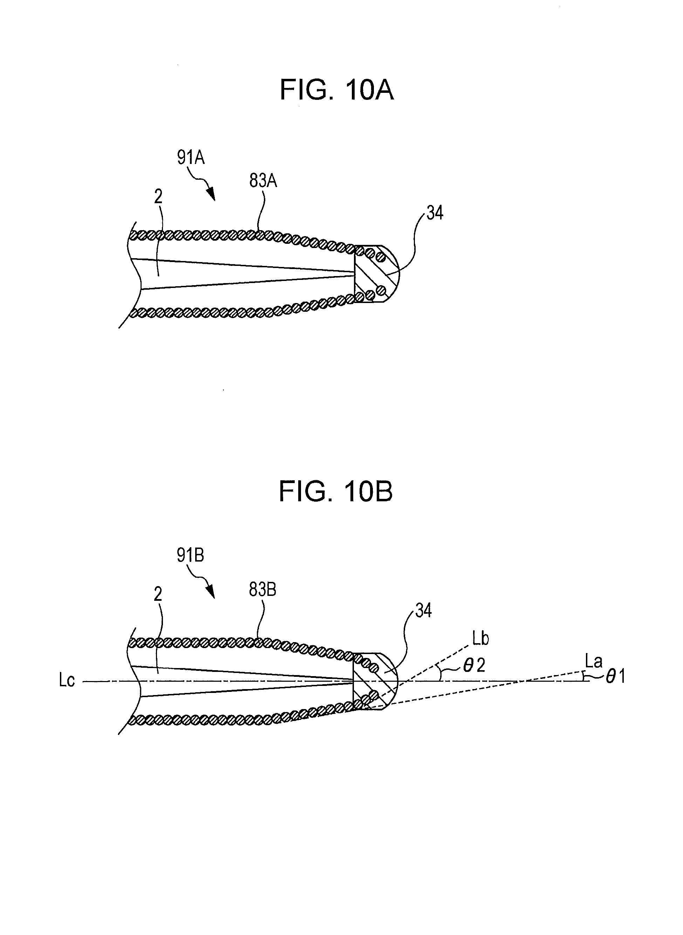

Fig. 10A illustrates a guidewire 91A including a tapered coil body 83A. A part of the tapered coil body 83A in the proximal direction of a most distal end portion 34 has, instead of a straight shape, a tapered shape having a coil outer diameter that decreases in the distal direction. The taper angle of a part of the tapered coil body 83A in the most distal end portion 34 is the same as the taper angle of the tapered coil body 83A at the proximal end of the most distal end portion 34.

Fig. 10B illustrates a guidewire 91B including a tapered coil body 83B. In a part of the tapered coil body 83B in the proximal direction of the most distal end portion 34, the angle between the extension line La of the taper coil and the central axis Lc of the guidewire is θ1. The taper angle of the tapered coil body 83B is changed at the proximal end of the most distal end portion 34, and the angle between the extension line Lb of the taper coil and the central axis Lc is θ2 (θ2 > θ1). In this modification, θ2 is larger than θ1. However, these angles are not limited thereto, and may be θ1 > θ2.

The modification illustrated in Fig. 10B is made to a tapered coil body. However, the modification can be applied to a straight coil body. For example, in the first embodiment illustrated in Fig. 1, the decreasing-diameter shape (tapered shape) of a part of the coil body 3 in the most distal end portion 4 may be changed at the middle of the most distal end portion 4. This method is applicable not only to a decreasing-diameter shape but also to an increasing-diameter shape.

In Fig. 10B, the angle changes between two levels. However, this is not limited thereto, and the angle may change among three or more levels.

The shapes of the coil bodies illustrated in Figs. 4 to 10 can be applied to the shape of each of an outer coil body and an inner coil body included in a guidewire having a double coil body structure as in the second or third

### embodiment.

### Eighth Modification

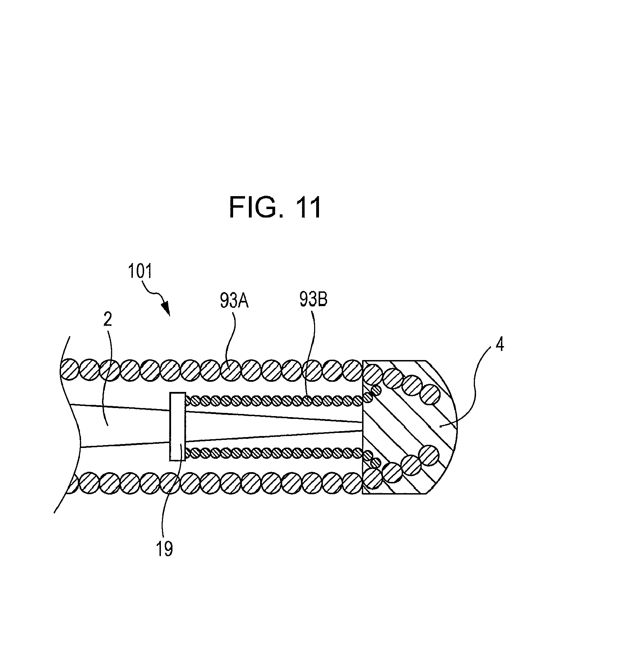

Fig. 11 illustrates a guidewire 101 including an outer coil body 93A and an inner coil body 93B. The coil outer diameter of a part of the outer coil body 93A in the most distal end portion 4 decreases in the distal direction. The coil outer diameter of a part of the inner coil body 93B in the most distal end portion 4 increases in the distal direction.

### Ninth Modification

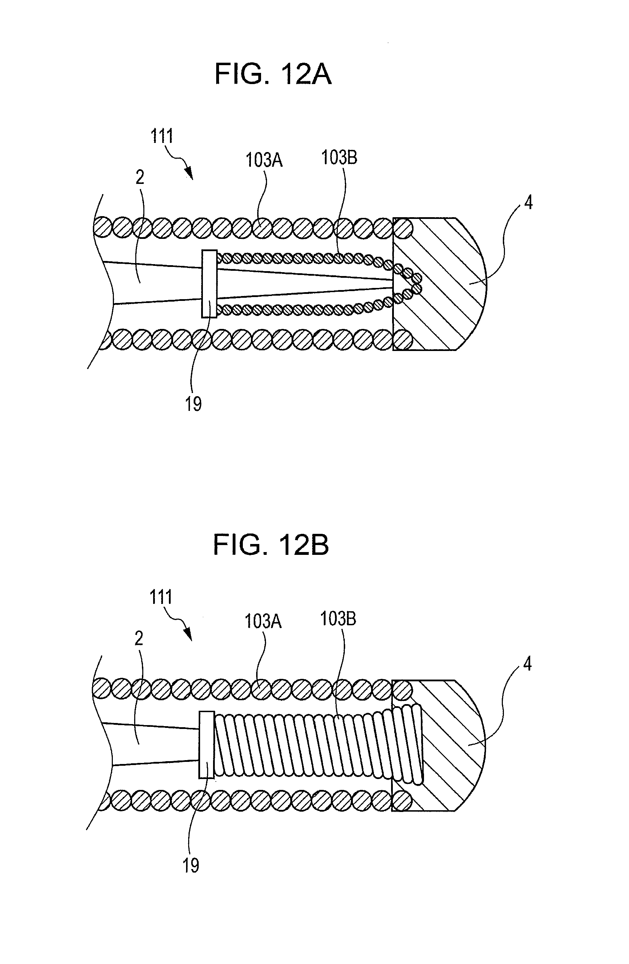

Fig. 12A illustrates a sectional view of a guidewire 111, and Fig. 12B illustrates the guidewire 111 of Fig. 12A rotated by 90 degrees around the axis of the guide wire 111. A part of an outer coil body 103A in the most distal end portion 4 and a part of the outer coil body 103A in the proximal direction of the most distal end portion 4 have a uniform coil outer diameter. A distal end portion of an inner coil body 103B is pressed so that the inner space of the inner coil body 103B narrows in the distal direction. That is, the distal end portion of the inner coil body 103B has an outer diameter (dimensions) such that the width thereof increases and the thickness thereof decreases in the distal direction.

The inner coil body 103B, which has such a shape, can be made by fixing the inner coil body 103B to the core shaft 2, and before fixing the outer coil body 103A to the core shaft 2, pressing the distal end portion of the inner coil body 103B. It is necessary that, when the inner coil body 103B has been fixed to the core shaft 2, the distal end of the inner coil body 103B protrude from the distal end of the outer coil body 103A in the distal direction. The inner coil body 103B may be made by pressing the protruding part of the inner coil body 103B. When pressing the inner coil body 103B, the distal end of the core shaft 2 may be pressed together with the inner coil body 103B.

In each of the modifications illustrated in Figs. 4 to 12, the coil outer diameter of a part of the coil body in the most distal end portion is nonuniform. Therefore, accidental removal of the most distal end portion from the guidewire can be prevented. Moreover, the structure for preventing accidental removal can be obtained by changing only the coil outer diameter of the coil body, so that flexibility of the guidewire near the distal end thereof is maintained.

Instead of a single wire coil, a multistrand coil made of a plurality of strands may be used as the coil body. By forming the coil body from a plurality of strands, the mechanical strength of the coil body is increased and accidental removal of the most distal end portion is further reliably prevented.

Although not illustrated, the coil body may be fixed to the core shaft not only at the proximal end of the coil body and at the most distal end portion but also at a middle part of the coil body by using a brazing alloy or the like. Such a brazed joint may be formed between the outer coil body and the inner coil body or between a middle part of the inner coil body and the core shaft.

With such a structure, accidental removal of the entirety of the most distal end portion and the coil body is prevented.

The outer diameter of the coil body may be made nonuniform by using a method of, for example, heating the coil body or the strand beforehand so that the shape of the coil body can be easily made nonuniform.

When making a coil body having an outer diameter that is nonuniform, the shape of the coil body can be stably maintained by heat-treating the coil body after changing the outer diameter of the coil body using a die or the like.

In the above-described embodiments and modifications, a double coil structure is used as an example of a multi-coil structure. However, this is not limited thereto, and a structure having three or more coils may be used for the guidewire as long as the flexibility of the guidewire can be maintained.

When forming the most distal end portion from a brazing alloy or the like, a flux may be applied to the distal end portion of the core shaft and to the distal end portion of the coil body so as to increase the wettability of the distal end portions with the brazing alloy. Moreover, plating for increasing the wettability with the brazing alloy may be performed on the distal end portion of the core shaft and the distal end portion of the coil body.

The present invention contains subject matter related to Japanese Patent Application No. 2010-173603 filed in the Japan Patent Office on August 2, 2010, the entire contents of which are incorporated herein by reference.

## Claims

1. A guidewire (1; 11; 21; 31; 41; 51; 61; 71; 81;
91A; 91B; 101; 111) comprising:
a core shaft (2);
at least one coil body (3; 13A; 23; 33; 43; 53; 63; 73; 83A; 83B; 93A; 103A) wound around an outer periphery of the core shaft (2); and
a most distal end portion (4; 14; 24; 34) formed by fixing a distal end portion of the core shaft (2) and a distal end portion of the at least one coil body (3; 13A; 23; 33; 43; 53; 63; 73; 83A; 83B; 93A; 103A) to each other, **characterized in that**
the coil outer diameter of at least one coil body (3; 13A; 23; 33; 43; 53; 63; 73; 83A; 83B; 93A; 103A) in the most distal end portion (4; 14; 24; 34) is nonuniform.

2. The guidewire (1; 11; 21; 31; 41; 51; 71; 81; 91A;
91B; 101) according to Claim 1,
wherein the coil outer diameter of the coil body (3;
13A; 23; 33; 43; 63; 73; 83A; 83B; 93A) in the most distal end portion (4; 24; 34) decreases in a distal direction.

3. The guidewire (11; 21; 101; 111) according to claim 1, wherein the at least one coil body (13A; 13B; 13C; 93A;
93B; 103A; 103B) comprises:
an outer coil body (13A; 93A; 103A) wound around an outer periphery of the core shaft (2); and
an inner coil body (13B; 13C; 93B; 103B) located inside the outer coil body (13A; 93A; 103A) and wound around an outer periphery of the core shaft (2);
wherein the most distal end portion (4) is formed by fixing a distal end portion of the core shaft (2), a distal end portion of the outer coil body (13A; 93A; 103A), and a distal end portion of the inner coil body (13B; 13C; 93B; 103B) to one another, and
wherein at least one of a coil outer diameter of the outer coil body (13A; 93A; 103A) and a coil outer diameter of the inner coil body (13B; 13C; 93B; 103B) in the most distal end portion (4) is nonuniform.

4. The guidewire (11; 21; 101; 111) according to Claim 3,
wherein the coil outer diameter of the outer coil body (13A; 93A) in the most distal end portion (4) decreases in a distal direction.
